# EUROPEAN PATENT APPLICATION

(11) **EP 3 928 658 A1**
(43) Date of publication of application: **29.12.2021**
(21) Application number: 20759194.2
(22) Date of filing: 09.01.2020
(51) Int. Cl.: A45D 44/22, B32B 27/00, A61K 8/02

(54) **COSMETIC LAYERED SHEET, METHOD FOR MANUFACTURING SAME, COSMETIC SET, AND METHOD FOR ATTACHING THIN FILM SHEET**

(30) Priority: 22.02.2019 JP 2019030612
(71) Applicant: Panasonic Intellectual Property Management Co., Ltd., Osaka-shi, Osaka 540-6207 (JP)
(72) Inventor: UEDA, Mari, Osaka-shi, Osaka 540-6207 (JP); SHINODA, Masayo, Osaka-shi, Osaka 540-6207 (JP); TAKESHITA, Sachiko, Osaka-shi, Osaka 540-6207 (JP)
(74) Representative: Grünecker Patent- und Rechtsanwälte PartG mbB
(86) International application number: PCT/JP2020/000416
(87) International publication number: WO 2020/170633

(57) **Abstract**

A cosmetic layered sheet (100) includes a hydrophilic substrate (11), a thin film sheet having liquid permeability and laminated on the hydrophilic substrate (12), and a support (13) laminated on the thin film sheet. The thin film sheet is impregnated with a liquid containing a hydrophilic component, a lipophilic component, and a surfactant having an HLB value of 3 or more, and the contact angle of the hydrophilic substrate with water is smaller than that of the material constituting the support with water.

## Description

### Technical Field

The present disclosure relates to a cosmetic layered sheet and a method for manufacturing the same, a cosmetic set, and a method for attaching a thin film sheet.

### Background Art

It has been proposed that an ink containing various coloring materials is applied to a thin film, and the film is attached to a human body to make a spot, a bruise, or a scar (hereinafter, also referred to as "discoloration area") on the skin less noticeable (for example, PTL 1). In the technology of PTL 1, the skin is imaged to identify the discoloration area. Then, a color similar to the color in the periphery of the discoloration area is printed on a thin film sheet, and the sheet is attached to the skin to make the discoloration area less noticeable.

Such a thin film sheet is usually distributed by being laminated on a mount, and is peeled off from the mount by a user and attached to the skin. However, the thin film sheet is very thin and is therefore difficult to be attached to the skin without causing wrinkles. Accordingly, PTL 2 proposes an attaching method using a jig. Specifically, a layered product in which a mount and a thin film sheet are laminated is placed on a jig such that the thin film sheet and the jig face each other. Water is then sprayed on the mount side. The mount is then peeled off, the exposed thin film sheet is closely attached to the skin, and the jig is peeled off from the thin film sheet.

### Citation List

### Patent Literature

PTL 1: Japanese Unexamined Patent Application Publication No. 2015-43836
PTL 2: International Publication No. WO 2018/061486

### Summary of Invention

Attachment of a thin film sheet to a surface for attachment (e.g., skin) is easy by using a jig as the method of PTL 2. However, since the thin film sheet is very thin, it is difficult to peel off the amount from the thin film sheet. For example, when the mount is peeled off, the thin film sheet also rises up to peel off from the jig or cause wrinkles on the thin film sheet in some cases. If wrinkles occur on the thin film sheet on the jig, wrinkles are likely to occur also on the thin film sheet attacked to skin.

The present disclosure provides a cosmetic layered sheet in which a support is easily peeled off and also wrinkles are unlikely to occur on a thin film sheet after peeling of the support, and a method for manufacturing the cosmetic layered sheet. The cosmetic layered sheet of the present disclosure is composed of a hydrophilic substrate, a thin film sheet having liquid permeability and laminated on the hydrophilic substrate, and a support laminated on the thin film sheet. The thin film sheet is impregnated with a liquid containing a hydrophilic component, a lipophilic component, and a surfactant having an HLB value of 3 or more, and the cosmetic layered sheet is configured such that the contact angle of the hydrophilic substrate with water is smaller than the contact angle of the material constituting the support with water.

According to a cosmetic set of the present disclosure, the support is easily peeled off from the thin film sheet. In addition, wrinkles are unlikely to occur on the thin film sheet after peeling of the support.

### Brief Description of Drawings

[Fig. 1] Fig. 1 is a schematic cross-sectional view of a cosmetic layered sheet according to an embodiment of the present disclosure.
[Fig. 2A] Fig. 2A is a schematic diagram for explaining a method for peeling off a support from a known cosmetic layered sheet.
[Fig. 2B] Fig. 2B is a schematic diagram for explaining a method for peeling off a support from a cosmetic layered sheet according to an embodiment of the present disclosure.
[Fig. 3] Fig. 3 is a schematic cross-sectional view of a cosmetic set according to a first embodiment of the present disclosure.
[Fig. 4] Fig. 4 is a schematic cross-sectional view of a cosmetic set according to a second embodiment of the present disclosure.
[Fig. 5] Fig. 5 is a schematic cross-sectional view of a cosmetic set according to a third embodiment of the present disclosure.

### Description of Embodiments

### 1. Cosmetic layered sheet

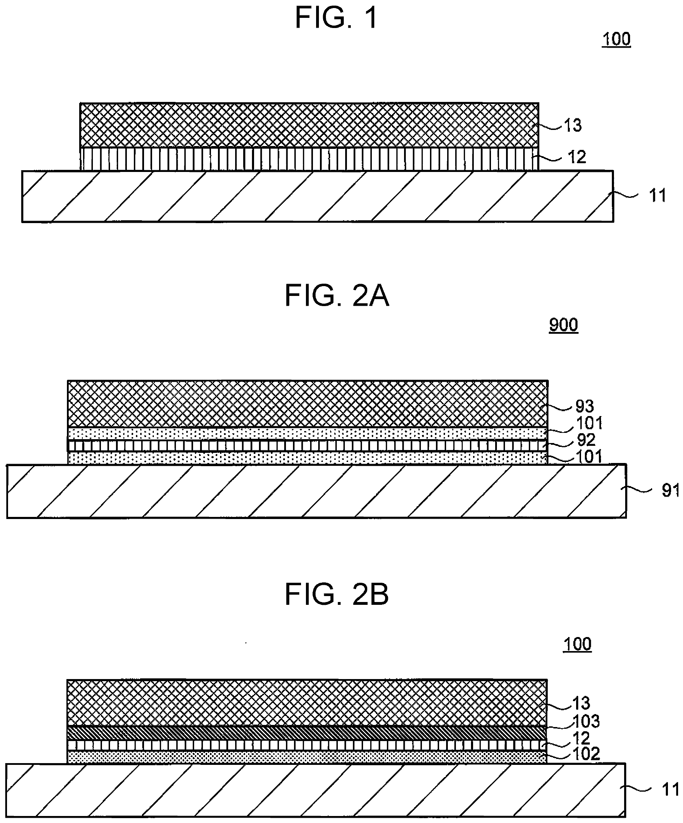

Fig. 1 shows a cross-sectional view of a cosmetic layered sheet according to an embodiment of the present disclosure. The cosmetic layered sheet of the present disclosure at least includes a hydrophilic substrate 11, a thin film sheet 12 that is arranged on the hydrophilic substrate 11 and to be attached to skin for coloring or beautification of the skin, and a support 13 for supporting them. In the cosmetic layered sheet 100, the thin film sheet 12 is impregnated with a liquid containing a hydrophilic component, a lipophilic component, and a surfactant having an HLB value of 3 or more (not shown).

The thin film sheet 12 of the cosmetic layered sheet 100 may be a sheet for being used by a specific person (sheet produced on demand) produced according to a discolored portion of the skin of the specific individual by, for example, a known makeup support system. Alternatively, the thin film sheet 12 may be, for example, a sheet for being used by a large number of unspecified persons produced to match the average skin color of a large number of unspecified persons. The thin film sheet 12 can be used not only for coloring or the like of skin but also in application for making a discolored portion less noticeable or for decorating the discolored portion by being attached to the discolored portion of the skin.

Fig. 2A shows a schematic view for explaining a method for peeling off a support from a known cosmetic layered sheet. It has been proposed that a cosmetic layered sheet 900 composed of a hydrophilic substrate 91, a thin film sheet 92, and a support 93 laminated in this order is sprayed with water from the support 93 side to peel off the support 93.

The support 93 and the thin film sheet 92 absorb water by spraying water to the cosmetic layered sheet 900 to form a water layer 101 between the thin film sheet 92 and the support 93 and between the thin film sheet 92 and the hydrophilic substrate 91, respectively. Incidentally, in Fig. 2A, the water layer 101 is drawn to have a large thickness for convenience, but is actually a very thin layer. It is inferred that the formation of the water layer 101 between the thin film sheet 92 and the support 93 separates the thin film sheet 92 and the support 93 from each other to make the support 93 be easily peeled off. However, in this method, lamination adsorption force occurs due to hydration by the water layer 101 between the thin film sheet 92 and the support 93 and between the thin film sheet 92 and the hydrophilic substrate 91. In addition, there is no significant difference between the lamination adsorption force occurring between the thin film sheet 92 and the support 93 and the lamination adsorption force occurring between the thin film sheet 92 and the hydrophilic substrate 91. Accordingly, when the support 93 is peeled off from the thin film sheet 92, peeling occurred not at the interface between the support 93 and the thin film sheet 92 but at the interface between the thin film sheet 92 and the hydrophilic substrate 91 in some cases.

Fig, 2B shows a schematic view for explaining a method for peeling off a support from a cosmetic layered sheet 100 of the present disclosure. In the cosmetic layered sheet 100 of the present disclosure, the thin film sheet 12 is impregnated with a liquid containing a hydrophilic component, a lipophilic component, and a surfactant having an HLB value of 3 or more. In addition, in the cosmetic layered sheet 100 of the present disclosure, the contact angle of the hydrophilic substrate 11 with water is smaller than the contact angle of the material constituting the support 13 with water. That is, the compatibility of the hydrophilic substrate 11 with the hydrophilic component is higher than that of the support 13, and the compatibility of the support 13 with the lipophilic component is higher than that of the hydrophilic substrate 11. Accordingly, the hydrophilic component in the liquid impregnated in the thin film sheet 12 is more attracted to the hydrophilic substrate 11 side, and a layer containing a large amount of the hydrophilic component (hereinafter, also referred to as "hydrophilic layer") 102 is formed between the thin film sheet 12 and the hydrophilic substrate 11. In contrast, between the thin film sheet 12 and the support 13, the amount of the hydrophilic component is relatively small, and the concentration of the lipophilic component is high. That is, a layer containing a relatively large amount of the lipophilic component (hereinafter, also referred to as "lipophilic layer") 103 is formed between the thin film sheet 12 and the support 13. Incidentally, in Fig. 2B, the hydrophilic layer 102 and the lipophilic layer 103 are drawn to have large thicknesses, but they are actually very thin layers.

Here, the hydrophilic component contained in the hydrophilic layer 102 in a large amount easily forms hydrogen bonds with the hydrophilic substrate 11 and the thin film sheet 12. In contrast, the lipophilic component contained in the lipophilic layer 103 in a large amount is unlikely to form such hydrogen bonds. Accordingly, the lamination adsorption force occurring between the thin film sheet 12 and the hydrophilic substrate 11 is higher than the lamination adsorption force occurring between the thin film sheet 12 and the support 13. As a result, when the support 13 is lifted, peeling is unlikely to occur at the interface between the thin film sheet 12 and the hydrophilic substrate 11, and the support 13 can be reliably peeled off from the thin film sheet 12.

Incidentally, the cosmetic layered sheet 100 of the present disclosure also has advantages that when the thin film sheet 12 is attached to a surface for attachment, gloss is easily caused on the surface of the thin film sheet 12 by the lipophilic component in the liquid, and the aesthetic properties after attachment of the thin film sheet 12 easily become good. Each configuration of the cosmetic layered sheet of the present disclosure will now be described.

### (Hydrophilic substrate)

The hydrophilic substrate 11 included in the cosmetic layered sheet 100 of the present embodiment is a substrate that has hydrophilicity and a contact angle with water lower than that of the material constituting the support 13 described later. In the present specification, a substrate having hydrophilicity is a substrate having a contact angle with water of 90° or less. The contact angle of the hydrophilic substrate 11 with water is preferably 90° or less and more preferably 50° or less. In addition, the difference between the contact angle of the hydrophilic substrate 11 with water and the contact angle of the material constituting the support 13 described later with water is preferably 10° or more and more preferably 40° or more. When the difference between these contact angles is within this range, the hydrophilic component in the solution described later easily moves to the hydrophilic substrate 11 side, and the above-described effects are easily obtained.

In the present embodiment, the planar view shape of the hydrophilic substrate 11 is not particularly limited as long as it is larger than the planar view shape of the thin film sheet 12. The hydrophilic substrate 11 may be tabular or may have a curved surface (concave or convex). In addition, the thickness thereof is not particularly limited and is preferably about 0.1 to 15 mm and further preferably about 0.1 to 8 mm from the viewpoint of handling.

Furthermore, the hydrophilic substrate 11 may have a configuration (e.g., a screw thread provided on the side surface) for being joined with a container, a protection member, etc. or a convex or concave part or the like for position adjustment to them, when formed into a cosmetic set described later. In addition, a cutoff line may be formed at a position surrounding the thin film sheet 12 such that the thin film sheet 12 is easily taken out from the cosmetic set.

The hydrophilic substrate 11 may have or not have transparency, and preferably has transparency from the viewpoint of easily visually recognizing the thin film sheet 12 when the thin film sheet 12 is attached to a surface for attachment.

Examples of the hydrophilic substrate 11 include a sheet containing rayon, polyester, aramid, glass fiber, nylon, vinylon, polyolefin (e.g., polyethylene, polypropylene, or low density polyethylene), ethylene vinyl acetate resin, synthetic rubber, copolymerized polyamide resin, or copolymerized polyester resin; a silicon (Si)-based sheet containing a hydrophilic agent; an elastomer sheet containing a hydrophilic agent; and a rubber sheet made from aqueous gel. These sheets may be those subjected to various hydrophilization treatments.

### (Thin film sheet)

The thin film sheet 12 is a sheet for being attached to skin and has liquid permeability and is impregnated with a liquid described later. In the present specification, the phrase that a thin film sheet 12 has liquid permeability refers to that the hydrophilic component, the lipophilic component, and so on in a liquid described later can move from one surface of the thin film to the other. In addition, the phrase that a thin film sheet 12 is impregnated with a liquid refers to that both surfaces of the thin film sheet 12 are wet with a liquid described later.

The thin film sheet 12 can be a sheet composed of, for example, a thin film (not shown) having liquid permeability and a colored layer, a light scattering layer, or the like (not shown). When the thin film sheet 12 includes a colored layer, a light scattering layer, or the like in addition to the thin film, it is possible to color a surface for attachment (e.g., skin) to an arbitrary color by attachment of the thin film sheet 12. More specifically, it is possible to color the skin or make a discolored portion of skin look normal. Incidentally, when the thin film sheet 12 includes a colored layer or the like, the thin film sheet 12 is preferably attached such that the thin film comes into contact with a surface for attachment. When the surface for attachment is the skin of a human body, irritation etc. is less likely to occur in the skin by attaching a thin film having biocompatibility to the skin. Accordingly, when the thin film sheet 12 includes a colored layer, the thin film sheet 12 is laminated to a support 13 described later such that the thin film faces the support 13.

The thin film included in the thin film sheet 12 is preferably a sheet-like member that does not cause a sense of discomfort even when attached to human skin and has biocompatibility. In addition, the thin film is usually preferably colorless and transparent or translucent.

The thickness of the thin film in the thin film sheet 12 is preferably 10 nm to 10 µm and more preferably 10 to 1000 nm. In particular, when the thin film is hydrophobic, the thickness of the thin film is particularly preferably 10 to 800 nm.

The planar view shape of the thin film is not particularly limited, and is appropriately selected according to the shape of the portion where the thin film sheet 12 is attached and the application. Incidentally, the thin film may be provided with a slit in the circumference and/or in the inner area so as to fit to the shape of the portion where the thin film sheet 12 is attached.

Here, the thin film may be a sheet formed by a spin coating method, a roll-to-roll method, an LB method (Langmuir-Blodgett method), or the like or may be, for example, a fiber sheet in which fibers generated by an electrospinning method or the like overlap one another.

Examples of the material of the thin film include polyesters, typically, such as polyglycolic acid, polylactic acid, polycaprolactone, polyethylene succinate, polyethylene terephthalate, and copolymers thereof; polyethers, typically, such as polyethylene glycol and polypropylene glycol; polyamides, typically, such as nylon, polyglutamic acid, polyaspartic acid, and salts thereof; polysaccharides, typically, such as pullulan, cellulose, starch, chitin, chitosan, alginic acid, hyaluronic acid, and cornstarch and salts thereof; silicones, typically, such as acrylic silicone and trimethylsiloxysilic acid; acrylic acids, typically, such as alkyl acrylate, silicone acrylate, acrylamide, and copolymers thereof; polyvinyl alcohol; polyurethane; polycarbonate; polyacid anhydride; polyethylene; polypropylene; and porous layer coating sheets and nanofiber sheets. Among these materials, when the material of the thin film is polylactic acid, cellulose (e.g., carboxymethyl cellulose or hydroxyethyl cellulose), starch, chitin, chitosan, alginic acid, cornstarch, or polyurethane, for example, biocompatibility, availability, and handleability are improved.

On the other hand, the colored layer arranged on the thin film contains a coloring material and a binder. The colored layer may further contain, for example, a film-forming agent, a dispersant, and various additives. The color of the colored layer can be usually a color that matches the skin, but may be any color when the thin film sheet 12 is used as a cosmetic product, such as rouge, eyeshadow, or body painting. In addition, the whole thin film sheet 12 may be in a single color, or regions of different colors may be arranged partially.

In addition, the colored layer may be composed of one layer or may be composed of two or more layers. When the colored layer is composed of a plurality of layers, the types of the coloring materials contained in the respective layers may be the same or different. In addition, the amounts of the coloring materials contained in the respective layers may be the same or different. For example, the colored layer may be composed of a flesh-colored layer and an arbitrarily colored layer laminated thereon.

Examples of the coloring material contained in the colored layer include inorganic red pigments, for example, iron titanate, such as iron oxide and iron hydroxide; inorganic brown pigments, such as y-iron oxide; inorganic yellow pigments, such as yellow iron oxide and ocher clay; inorganic black pigments, such as black iron oxide and carbon black; inorganic purple pigments, such as manganese violet and cobalt violet; inorganic green pigments, such as chromium hydroxide, chromium oxide, cobalt oxide, and cobalt titanate; inorganic blue pigments, such as dark blue (ferric ferrocyanide), ultramarine blue, lapis lazuli, mountain blue, aluminum-cobalt oxide, aluminum-zinc-cobalt oxide, silicon-cobalt oxide, silicon-zinc-cobalt oxide, cobalt pigment, smalt, cobalt blue, cobalt stannate, cobalt chromium blue, cobalt-aluminum-silicon oxide, and manganese blue; organic blue pigments or blue dyes, such as indigo, phthalocyanine, indanthrene blue, and sulfonates thereof; and those obtained by laking various tar dyes, those obtained by laking various natural dyes, and composite synthetic resin powders thereof.

The shape of the binder contained in the colored layer is not particularly limited, and the binder is preferably particulate and is particularly preferably particles made of a (meth)acrylic resin (hereinafter, also simply referred to as "acrylic particles"). When the binder consists of acrylic particles, the fixability of the coloring material tends to be good, and the durability of the colored layer tends to be good. The binder is further preferably particles made of a (meth)acrylic resin not causing skin irritation. Accordingly, the acrylic particles are preferably selected from, for example, components listed in the component name list of cosmetics based on the Japanese Pharmaceutical Affairs Act, components complying with the EU Cosmetics Regulation (Cosmetics Directive 76/768/EEC), and components described in the International Cosmetic Ingredient Dictionary and Handbook (9th edition, January 1, 2002) by the U.S. CTFA (Cosmetic, Toiletry & Fragrance Association, U.S.), and are preferably particles of an acrylic resin that is applied to known cosmetics and so on.

The amount of the binder contained in the colored layer is preferably 0.5 to 10 parts by mass and more preferably 1.5 to 5.7 parts by mass based on 10 parts by mass of the color material. When the amount of the binder relative to the amount of the coloring material is within the range above, the fixability of the coloring material is enhanced. In addition, when the amount of the binder is within the range above, the amount of the coloring material tends to be relatively sufficient, and the colored layer can be made to have a desired color.

The thickness of the colored layer is appropriately selected in accordance with the desired color density, etc. and is preferably 10 nm to 15 µm and more preferably 10 nm to 3 µm. When the thickness of the colored layer is within this range, desired color development is easily obtained without causing a thick coating feeling. Incidentally, when a plurality of colored layers are laminated, the total thickness of them is preferably within the range above. Such a colored layer can be formed by applying an ink containing a coloring material and a binder onto a thin film by, for example, an ink jet printing method, a screen printing method, off-set printing, or gravure printing. Among these methods, the ink jet method is preferable from the viewpoint of, for example, easily performing on-demand printing and being capable of performing lamination printing by applying a cosmetic ink multiple times.

On the other hand, the light scattering layer contains a reflective material and a binder. The light scattering layer may further contain, for example, a film-forming agent, a dispersant, and various additives. The light scattering layer may be composed of one layer or may be composed of two or more layers. When the light scattering layer is composed of a plurality of layers, the types of the reflective materials contained in the respective layers may be the same or different. In addition, the amounts of the reflective materials contained in the respective layers may be the same or different.

The reflective material contained in the light scattering layer may be particles that scatter or reflect UV light and visible light (e.g., light with a wavelength of 200 to 780 nm), and can be, for example, a pearl agent, a soft focus agent, or a lame agent. The pearl agent, the soft focus agent, and the lame agent are preferably those not causing skin irritation.

The amount of the binder contained in the light scattering layer is preferably 0.5 to 10 parts by mass and more preferably 1.5 to 5.7 parts by mass based on 10 parts by mass of the reflective material. When the amount of the binder relative to the amount of the reflective material is within the range above, the fixability of the reflective material is enhanced. In addition, when the amount of the binder is within the range above, the amount of the reflective material tends to be relatively sufficient, and it is possible to sufficiently reflect or scatter light by the light scattering layer.

The thickness of the light scattering layer is preferably 10 nm to 120 µm and more preferably 10 nm to 100 µm. When the thickness of the light scattering layer is within this range, the light reflected by the surface of skin tends to be sufficiently reflected by the light scattering layer.

Incidentally, a glossy layer, a moisture-absorbing layer, or the like may be further laminated on the thin film sheet 12 within a range that does not impair the purpose and the effect of the present embodiment. When the moisture-absorbing layer is arranged, the moisture on the surface side of the thin film sheet 12 is controlled to increase comfort. The moisture-absorbing layer usually contains a moisture absorbent, and examples of the moisture absorbent include spherical silica, porous acrylic particles, and nylon 6 (polyamide 6).

### (Support)

The support 13 is a layer for protecting the above-described thin film sheet 12, and the shape thereof is not particularly limited as long as it can cover one of surfaces of the thin film sheet 12. The planar view shape of the support 13 may be the same as that of the thin film sheet 12 or may be larger than that of the thin film sheet 12. For example, the support 13 may have a tab to be gripped when peeled off from the thin film sheet 12.

In addition, the thickness of the support 13 is preferably 50 to 2000 µm and more preferably 100 to 1500 µm. When the thickness of the support 13 is within this range, when the support 13 is peeled off from the thin film sheet 12, the support 13 is easily deformed in an arbitrary direction and is easily peeled off.

The support 13 is constituted of a material having hydrophilicity or a material having water absorbability capable of retaining water inside. As described above, in the present specification, the material having hydrophilicity is a material having a contact angle of 90° or less, and the material having water absorbability is a material having a structure (e.g., hole) that can retain water inside. Incidentally, when the support 13 is constituted of a material having water absorbability, the contact angle of the material with water may exceed 90°. Here, the contact angle of the material constituting the support 13 with water is larger than the contact angle of the hydrophilic substrate 11 with water, as described above. The contact angle of the material constituting the support 13 with water is preferably 150° or less and more preferably 145° or less. The contact angle of the material constituting the support 13 with water may be determined by producing a tabular sample made of the material and measuring the contact angle of the sample with water.

Examples of the material of the support 13 include paper (cellulose), rayon, polyester, aramid, glass fibers, nylon, vinylon, polyolefins (e.g., polyethylene, low density polyethylene, and polypropylene), ethylene vinyl acetate resin, synthetic rubber, copolymerized polyamide resin, copolymerized polyester resin, porous films, and nanofiber sheets. Among these materials, paper (cellulose), rayon, and polyester are preferable from the viewpoint of easily being peeled off from the thin film sheet 12. In addition, the support 13 may have or not have optical transparency or may be colored.

In addition, the support 13 preferably has flexibility, and the elastic modulus thereof is preferably 100 to 100000 MPa and more preferably 100 to 5000 MPa. The elastic modulus is a value measured by any of a static test method, a transverse vibration method, and an ultrasonic method. The JIS standards for elastic modulus measurement are JIS Z2280, JIS R1602, and JIS R1605, and the elastic modulus is a value measured in conformity to any of them. The support 13 having an elastic modulus within the range above is, for example, unlikely to be ruptured and can be appropriately deformed when being peeled off from the thin film sheet 12.

### (Liquid)

The liquid impregnated in the thin film sheet 12 described later is a liquid containing a hydrophilic component, a lipophilic component, and a surfactant. The HLB value of the surfactant may be 3 or more and is preferably 3 to 20 and more preferably 6 to 20.

The liquid can be an emulsion in which one of a hydrophilic component and a lipophilic component is dispersed in the other. The liquid may be an oil-in-water type (O/W type) emulsion or may be a water-in-oil type (W/O type) emulsion and is preferably an oil-in-water type (O/W type) emulsion from the viewpoint of, for example, easily fitting to the skin.

Examples of the hydrophilic component include various alcohols and water, and among these examples, preferred are water and alcohols having 3 or less carbon atoms. Alcohols having 3 or less carbon atoms have high water solubility.

Examples of the lipophilic component include mineral oil (such as paraffin, mineral oil, squalene, and Vaseline), vegetable oil, animal oil, synthetic fat (such as olefin oligomer and silicone oil), fatty acid esters, lanolin and derivatives thereof, aliphatic higher alcohols, phospholipids, and fatty acids. These components are highly effective in retaining moisture and barrier function of the skin.

In addition, the surfactant is not particularly limited as long as the mixed state of the hydrophilic component and the lipophilic component can be stabilized, and examples thereof include anionic surfactants, cationic surfactants, amphoteric surfactants, and nonionic surfactants. Among these surfactants, amphoteric surfactants and nonionic surfactants are preferred from the viewpoint of low irritation and toxicity to skin and of providing stable emulsifying force. These surfactants are more preferably components listed in the component name list of cosmetics based on the Japanese Pharmaceutical Affairs Act, components complying with the EU Cosmetics Regulation (Cosmetics Directive 76/768/EEC), and components described in the International Cosmetic Ingredient Dictionary and Handbook (9th edition, January 1, 2002) by the U.S. CTFA (Cosmetic, Toiletry & Fragrance Association, U.S.). In particular, polyethylene glycol has significantly high compatibility with water and is therefore particularly preferable.

The amount of the hydrophilic component in the liquid is appropriately selected, and is preferably 98 mass% or less and more preferably 5 to 98 mass% in the liquid. In addition, the amount of the lipophilic component is preferably 98 mass% or less and more preferably 5 to 98 mass% in the liquid. At the same time, the amount of the surfactant is preferably 2 to 50 parts by mass and more preferably 2 to 10 parts by mass based on 100 parts by mass of the total amount of the hydrophilic component and the lipophilic component.

The viscosity of the liquid measured with a cone-plate viscometer at a shear velocity of 2000 (1/s) is preferably 1500 mPa·s or less and more preferably 1.2 to 15 mPa·s. When the viscosity of the liquid is within this range, the liquid tends to stay in the vicinity of the thin film sheet 12.

The method for preparing the liquid is not particularly limited, and can be the same as a known method for manufacturing an oil-in-water or water-in-oil type cosmetic.

### 2. Method for manufacturing cosmetic layered sheet

Regarding the method for manufacturing the cosmetic layered sheet, two embodiments will now be described, but the method for manufacturing the cosmetic layered sheet is not limited to these methods.

### [First Embodiment]

The method for manufacturing a cosmetic layered sheet of a First Embodiment includes a step of preparing a layered product in which the thin film sheet 12 and the support 13 are laminated (hereinafter, also referred to as "layered product preparation step"), a step of preparing the hydrophilic substrate 11 and laminating the hydrophilic substrate 11 to the layered product produced earlier so as to face the thin film sheet 12 (hereinafter, also referred to as "hydrophilic substrate lamination step"), and a step of applying a liquid containing a hydrophilic component, a lipophilic component, and a surfactant having an HLB value of 3 or more onto the support 13 (hereinafter, also referred to as "liquid application step"). However, the method for manufacturing a cosmetic layered sheet of the present embodiment may include another step as needed.

### (Layered product preparation step)

In the layered product preparation step, a layered product in which the thin film sheet 12 and the support 13 are laminated is prepared. In this step, a support 13 is prepared in advance, and a thin film sheet 12 may be formed on the support 13. Alternatively, a support 13 and a thin film sheet 12 produced separately may be stacked. Incidentally, when the thin film sheet 12 is a layered product composed of a thin film and a colored layer, a light scattering layer, or the like, the thin film is stacked so as to face the support.

In addition, the timing for forming the colored layer, the light scattering layer, and so on in the thin film sheet 12 is not particularly limited. For example, a colored layer or the like is formed on a thin film to provide a thin film sheet 12 (a layered product composed of a thin film and a colored layer or the like), and the product may be then laminated with a support 13. Alternatively, a thin film and a support 13 are laminated, and a colored layer or the like may be formed on the thin film.

### (Hydrophilic substrate lamination step)

In the hydrophilic substrate lamination step, the hydrophilic substrate 11 is prepared, and the layered product prepared in the layered product preparation step and the hydrophilic substrate are then stacked such that the hydrophilic substrate 11 faces the thin film sheet 12 of the layered product. Incidentally, no adhesion layer or the like is provided between these layers. A lamination adsorption force occurs between the layered product (thin film sheet 12) and the hydrophilic substrate 11 by applying a liquid from the support 13 side in the liquid application step described below, and the layers are closely attached to each other.

### (Liquid application step)

After the hydrophilic substrate lamination step, the liquid containing a hydrophilic component, a lipophilic component, and a surfactant having an HLB value of 3 or more is applied onto the support 13. The method for applying the liquid is not particularly limited as long as the liquid can be applied to sufficiently wet the support 13 and the thin film sheet 12. For example, the application can be performed by a known printing method, such as an ink jet printing method, a screen printing method, off-set printing, or gravure printing.

The liquid applied onto the support 13 permeates through the support 13 and moves to the thin film sheet 12 side. On this occasion, the hydrophilic component in the liquid is attracted and moves to the hydrophilic substrate 11 side to form the above-described hydrophilic layer between the thin film sheet 12 and the hydrophilic substrate 11. In contrast, the lipophilic component remains at the interface between the thin film sheet 12 and the support 13 to form the above-described lipophilic layer. As a result, when the support 13 is lifted, peeling easily occurs at the interface between the support 13 and the thin film sheet 12, and only the support 13 can be reliably peeled off.

### [Second Embodiment]

The method for manufacturing a cosmetic set of a Second Embodiment includes a step of preparing a layered product in which the thin film sheet 12 and the support 13 described above are laminated (hereinafter, also referred to as "layered product preparation step"), a step of preparing the above-described hydrophilic substrate 11 and applying the above-described liquid containing a hydrophilic component, a lipophilic component, and a surfactant having an HLB value of 3 or more onto at least one surface of the hydrophilic substrate 11 (hereinafter, also referred to as "liquid application step"), and a step of laminating the hydrophilic substrate 11 and the layered product in such a manner that the surface coated with the liquid of the hydrophilic substrate 11 faces the thin film sheet 12 (hereinafter, also referred to as "lamination step"). Incidentally, since the layered product preparation step is the same as the layered product preparation step of the First Embodiment, detailed explanation here is omitted. In addition, the method for manufacturing a cosmetic layered sheet of the present embodiment may include another step as needed.

### (Liquid application step)

In the liquid application step, the liquid is applied to at least one surface of the hydrophilic substrate 11. The method for applying the liquid in the liquid application step is not particularly limited as long as a sufficient amount of the liquid can be applied. For example, the application can be performed by a known printing method, such as an ink jet printing method, a screen printing method, off-set printing, or gravure printing.

### (Lamination step)

The hydrophilic substrate 11 and the layered product are laminated such that the surface coated with the liquid of the hydrophilic substrate 11 by the liquid application step faces the thin film sheet 12 of the layered product prepared in the layered product preparation step. When the lamination step is performed, a part of the components of the liquid permeates through the thin film sheet 12 and moves to the support 13 side. On this occasion, many of the hydrophilic component in the liquid stays on the hydrophilic substrate 11 side. Accordingly, a hydrophilic layer containing a large amount of the hydrophilic component is formed between the thin film sheet 12 and the hydrophilic substrate 11, and a lipophilic layer containing a large amount of the lipophilic component is formed between the thin film sheet 12 and the support 13. As a result, when the support 13 is lifted, peeling easily occurs at the interface between the support 13 and the thin film sheet 12, and only the support 13 can be reliably peeled off.

### 3. Method for attaching thin film sheet

The method for attaching the containing the cosmetic layered sheet 100 to a surface for attachment will now be described. The method for attaching the thin film sheet 12 of the present disclosure performs a support peeling step of peeling off the support 13 from the cosmetic layered sheet 100 to expose the thin film sheet 12, and a thin film sheet attaching step of stacking the thin film sheet 12 to a surface for attachment and then peeling off the hydrophilic substrate 11 from the thin film sheet 12.

However, the method preferably further includes a water application step of applying water to a surface of the thin film sheet 12 or the surface for attachment (not shown) between the support peeling step and the thin film sheet attaching step.

### (Support peeling step)

As described above, in the cosmetic layered sheet of the present disclosure, the thin film sheet 12 is impregnated with the liquid, and the lamination adsorption force between the thin film sheet 12 and the hydrophilic substrate 11 is higher than the lamination adsorption force between the thin film sheet 12 and the support 13. Accordingly, when the support 13 is peeled off from the thin film sheet 12, the thin film sheet 12 is unlikely to be lifted, and only the support 13 can be reliably peeled off. In addition, in the cosmetic layered sheet 100, wrinkles or the like are unlikely to occur on the thin film sheet 12 after peeling of the support 13.

### (Water application step)

As described above, it is preferable to perform a water application step of spraying water to the surface of the thin film sheet 12 exposed by the support peeling step or to the surface for attachment to which the thin film sheet 12 is attached. When water is present on the surface of the thin film sheet 12 or the surface for attachment, the thin film sheet 12 and the surface for attachment are sufficiently closely attached to each other in the thin film sheet attaching step described below, and the hydrophilic substrate 11 tends to be easily peeled off from the thin film sheet 12.

The method for applying water is not particularly limited. For example, application can be performed by spraying. In addition, the amount is not particularly limited as long as the amount allows even application to the whole surface of the thin film sheet 12 or the whole surface for attachment.

### (Thin film sheet attaching step)

In the thin film sheet attaching step, the thin film sheet 12 is stacked to a surface for attachment, the hydrophilic substrate 11 is then peeled off from the thin film sheet 12, and only the thin film sheet 12 is transferred to the surface for attachment side. When the thin film sheet 12 is attached, the hydrophilic substrate 11 may be attached to, for example, a jig as needed. When a jig is used, the thin film sheet 12 is unlikely to be bent and is easily attached to a surface for attachment.

### (Others)

Incidentally, even if the cosmetic layered sheet 100 is used, for example, when the thin film sheet 12 is attached to a surface for attachment (e.g., skin) having a curved surface, there is a risk of causing wrinkles on the thin film sheet 12. On this occasion, it is preferable to stroke the wrinkled portion and the insufficiently attached portion with a brush or the like. The thin film sheet 12 can be closely attached to the surface for attachment or the wrinkles can be transported to the outer edge side of the thin film sheet 12 by stroking the surface of the thin film sheet 12 with a brush or the like. In addition, it is also possible to make the twisted end into a string and remove it.

The hardness of bristles of the brush or the like to be used is preferably 85 N/cm² or less and more preferably 60 N/cm² or less. When the hardness of bristles is within this range, wrinkles can be eliminated without damaging the thin film sheet 12. The hardness of bristles can be measured with a compression tester in conformity to JIS S3016 (1995).

In addition, the contact area of bristles of the brush or the like with the thin film sheet 12 is preferably 3 cm² or less. When the contact area is too broad, the risk of damaging the thin film sheet 12 is increased, further the pressure is dispersed to a broad area, and elimination of wrinkles or the like may be difficult.

Although the length of bristles of the brush or the like may be uniform, it is more preferable to include two or more types of bristles having different length. When bristles having different length are included, the points at which bristle ends are in contact with the thin film sheet 12 are minimized. Consequently, the friction occurring when the thin film sheet 12 is stroked with the bristles is suppressed, and damage of the thin film is likely to be suppressed. In this case, the difference between the lengths of the longest bristles and the shortest bristles of the brush or the like is preferably 1 to 10 mm and more preferably 1 to 5 mm.

### 4. Cosmetic set

The cosmetic set of the present disclosure includes the above-described cosmetic layered sheet and a container for supplying the above-described liquid onto the cosmetic layered sheet or a protection member. Regarding the cosmetic set, three embodiments will now be described, but the cosmetic set is not limited thereto.

### [First embodiment]

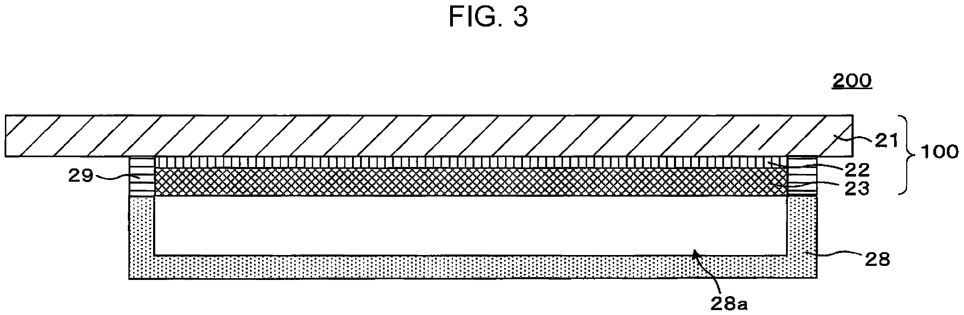

Fig. 3 shows a cross-sectional view of a cosmetic set according to a first embodiment of the present disclosure. As shown in Fig. 3, the cosmetic set 200 according to the present embodiment includes the cosmetic layered sheet 100 (hydrophilic substrate 21, thin film sheet 22, and support 23) and a container 28 joined to the support 23 side.

The container 28 arranged on the support side includes a concave part 28a having an opening that is substantially the same as or smaller than the support 23. In the cosmetic set 200, the cosmetic layered sheet 100 functions as a lid of the container 28 (concave part 28a). The container 28 contains the liquid.

The material of the container 28 is not particularly limited as long as it is not eroded by the liquid contained therein, and examples thereof include resin materials, such as polyethylene, polyethylene terephthalate, polypropylene, polyamide, polystyrene, vinyl chloride resin, AS resin, ABS resin, acrylic resin, and polycarbonate; materials made from natural materials such as sugar cane, reed, and kenaf; and metal materials, such as aluminum, stainless steel, tinplate, and steel. In addition, the shape is not particularly limited as long as it can contain a sufficient amount of the liquid, and, for example, the depth of the concave part 28a is appropriately selected.

The container 28 is joined to the hydrophilic substrate 21 with a joint 29. The joint 29 may be joined so as to surround the support 23 and the thin film sheet 22. The joint 29 may join the container 28 and the hydrophilic substrate 21 at, for example, two points or three or more points. The joint 29 is preferably constituted of a hydrophobic adhesive. The hydrophobic adhesive in the present specification is an adhesive made of a hydrophobic polymer and is hardly dissolved in water also after curing. Examples of the hydrophobic adhesive include various thermoplastic resin adhesives and thermosetting resin adhesives, and, more specifically, include a phenolic resin adhesive, a vinyl acetate adhesive, an epoxy resin adhesive, a cyanoacrylate adhesive, and a silicon adhesive.

In the cosmetic set 200, the liquid filled in the container 28 is supplied from the support 23 side, and the thin film sheet 22 is always impregnated with the liquid. When the thin film sheet 22 of the cosmetic set 200 is used, the hydrophilic substrate 21 is detached from the container 28. On this occasion, since the lamination adsorption force between the thin film sheet 22 and the hydrophilic substrate 21 is higher than the lamination adsorption force between the thin film sheet 22 and the support 23, peeling occurs at the interface between the thin film sheet 22 and the support 23. That is, the thin film sheet 22 can be peeled off together with the hydrophilic substrate 21. The thin film sheet 22 can be then attached to a surface for attachment by using the hydrophilic substrate 21 as a jig or the like.

### [Second embodiment]

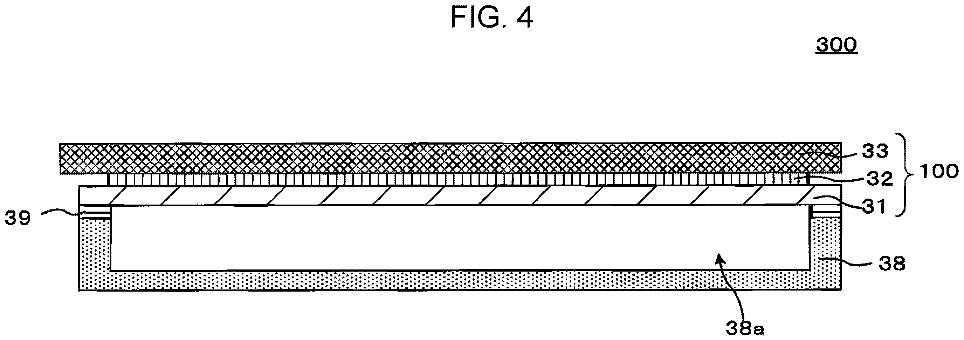

Fig. 4 shows a cross-sectional view of a cosmetic set according to a second embodiment of the present disclosure. As shown in Fig. 4, the cosmetic set 300 of the present embodiment includes a cosmetic layered sheet (support 33, thin film sheet 32, and hydrophilic substrate 31) and a container 38 arranged on the hydrophilic substrate 31 side.

The container 38 arranged so as to face the support includes a concave part 38a having an opening that is substantially the same as or smaller than the hydrophilic substrate 31. Also in the cosmetic set 300, the cosmetic layered sheet 100 functions as a lid of the container 38 (concave part 38a). The container 38 contains the liquid. The material and the shape of the container 38 can be the same as those of container 28 of the cosmetic set 200 of the first embodiment.

In addition, in the cosmetic set 300, the container 38 is joined to the hydrophilic substrate 31 with a joint 39. The joint 39 may join the container 38 and the hydrophilic substrate 31 so as to surround the circumference of the hydrophilic substrate 31. Alternatively, the joint 39 need not surround the circumference of the hydrophilic substrate 31, and may join the container 38 and the hydrophilic substrate 31 at, for example, two points or three or more points. The joint 39 is preferably constituted of a hydrophobic adhesive, and the same material as that of the joint 29 of the cosmetic set 200 of the first embodiment can be used.

In the cosmetic set 300, the liquid filled in the container 38 is supplied from the hydrophilic substrate 31 side, and the thin film sheet 32 is always impregnated with the liquid. When the thin film sheet 32 of the cosmetic set 300 is used, the support 33 is peeled off from the thin film sheet 32. On this occasion, since the lamination adsorption force between the thin film sheet 32 and the hydrophilic substrate 31 is higher than the lamination adsorption force between the thin film sheet 32 and the support 33, peeling reliably occurs at the interface between the thin film sheet 32 and the support 33. The thin film sheet 32 can be then attached to a surface for attachment by using the hydrophilic substrate 31 or the container 38 as a jig or the like.

### [Third embodiment]

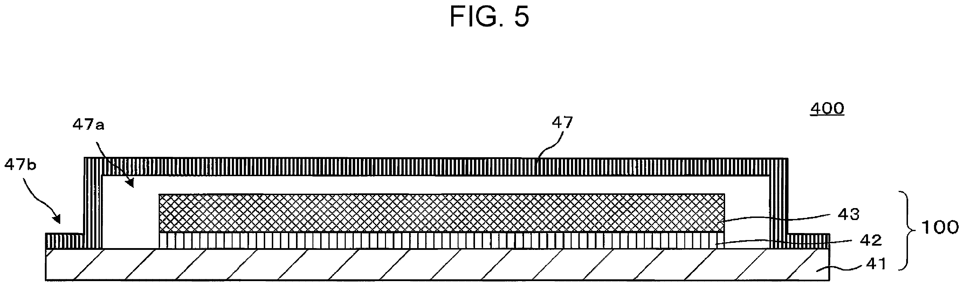

Fig. 5 shows a cross-sectional view of a cosmetic set according to a third embodiment of the present disclosure. As shown in Fig. 5, the cosmetic set 400 of the present embodiment includes a cosmetic layered sheet 100 (hydrophilic substrate 41, thin film sheet 42, and support 43) and a protection member 47 arranged so as to face the hydrophilic substrate 41 with the thin film sheet 42 and the support 43 of the cosmetic layered sheet 100 therebetween. The protection member 47 is joined to the hydrophilic substrate 41 so as to surround the thin film sheet 42 and the support 43 of the cosmetic layered sheet 100.

The protection member 47 may have any shape that covers the thin film sheet 42 and the support 43 and can join to the hydrophilic substrate 41. In the present embodiment, the protection member 47 includes a wall surface and a ceiling surface surrounding a concave part 47a for accommodating the thin film sheet 42 and the support 43 and a flange part 47b arranged so as to surround the opening edge of the concave part 47a. In the protection member 47, the concave part 47a accommodates the thin film sheet 42 and the support 43, and the flange part 47b and the hydrophilic substrate 41 are bonded to each other with an adhesive. However, the shape of the protection member 47 is not limited thereto and may be, for example, a flat sheet.

The material of the protection member 47 is not particularly limited as long as it is not eroded by the above-described liquid, and can be the same material as that of the container 28 of the cosmetic set 200 of the first embodiment. In addition, the adhesive for bonding the hydrophilic substrate 41 and the protection member 47 to each other is preferably a hydrophobic adhesive, and the same material as that of the joint 29 of the cosmetic set 200 of the first embodiment can be used.

Since the cosmetic set 400 is sealed by the protection member 47 and the hydrophilic substrate 41, the liquid impregnated in the thin film sheet 42 is unlikely to volatilize. Accordingly, the space defined by the hydrophilic substrate 41 and the protection member 41 need not be filled with a liquid, but the thin film sheet 42 can be stably impregnated with the liquid over a long period of time by filling the inside with the liquid.

When the cosmetic layered sheet 400 is used, the hydrophilic substrate 41, the thin film sheet 42, and the support 43 can be taken out by peeling off the protection member 47. The support 43 is then peeled off, and the thin film sheet 42 can be attached to a surface for attachment by using the hydrophilic substrate 41 as a jig or the like.

Incidentally, when a cutoff line is formed in the hydrophilic substrate 41, the thin film sheet 42, etc. may be taken out to the outside by cutting off a part of the hydrophilic substrate 41 along the cutoff line of the hydrophilic substrate 41.

In the description above, the hydrophilic substrate 41 and the protection member 47 are bonded to each other with an adhesive, but the method for joining the hydrophilic substrate 41 and the protection member 47 is not particularly limited. For example, a screw thread may be arranged on the side face of the hydrophilic substrate 41, and a thread part that can fit the screw thread may be provided to the protection member 47 side. In this case, the protection member 47 can be detached by rotating the protection member 47 in a certain direction. Alternatively, the hydrophilic substrate 41 and the protection member 47 may be sealed by sandwiching the fringe area of the hydrophilic substrate 41 and the flange part 47b of the protection member 47 by an elastic body, such as rubber, from above and below.

### EXAMPLES

The present disclosure will be described below with reference to examples. The scope of the present disclosure is not construed as limited by the examples.

### [Comparative Example]

A polylactic acid sheet (thin film sheet) having a thickness of 200 nm was attached to a support consisting of filter paper (contact angle with water: 35°) having a planar view shape larger than the polylactic acid sheet. The layered product was then placed on a hydrophilic substrate (contact angle with water: 10°). Water was then sprayed to the support side, the support protruding from the thin film sheet was grasped, and the support was lifted and peeled off from the thin film sheet.

### [Example 1]

The same manner as Comparative Example was performed except that a layered product consisting of a thin film sheet and a support was placed on a hydrophilic substrate, and a liquid containing water, an olefin oligomer, and polyethylene glycol 1000 (HLB value: 20) was then sprayed from the support side.

### [Example 2]

The same manner as Comparative Example was performed except that a liquid containing water, cyclopentasiloxane, and PEG-10 Dimethicone (HLB value: 4.5) was applied onto a hydrophilic substrate, and the thin film sheet of the layered product consisting of the thin film sheet and the support was stacked to the application surface.

### [Evaluation]

The ease of peeling when the support was peeled off was evaluated as follows. The results are shown in Table 1.
× : the support was hardly peeled off, and even if it could be peeled off, the thin film sheet wrinkled or was not peeled off cleanly.
O : the support could be easily peeled off, and the thin film sheet after peeling did not wrinkle.

**[Table 1]**

| | Liquid impregnated in thin film sheet | Ease of peeling of support |
|---|---|---|
| Comparative Example | Water | × |
| Example 1 | Emulsion (containing hydrophilic component, lipophilic component, and surfactant having an HLB value of 3 or more) | O |
| Example 2 | | O |

As shown in Table 1, the support was not peeled off in some cases when only water was applied, but when the contact angle of the hydrophilic substrate and water was smaller than the contact angle of the material constituting the support with water and the thin film sheet was impregnated with a liquid containing a hydrophilic component, a lipophilic component, and a surfactant having an HLB value of 3 or more, the detachability of the support was improved (Examples 1 and 2).

### Industrial Applicability

In the cosmetic layered sheet of the present disclosure, the support is easily peeled off from the thin film sheet, and further wrinkles are unlikely to occur on the thin film sheet after peeling of the support. Accordingly, the cosmetic layered sheet is very useful in application for making a discolored portion less noticeable or for decorating the discolored portion.

### Reference Signs List

11, 21, 31, 41, 91 hydrophilic substrate
12, 22, 32, 42, 92 thin film sheet
13, 23, 33, 43, 93 support
28, 38 container
47 protection member
100, 900 cosmetic layered sheet
200, 300, 400 cosmetic set

## Claims

1. A cosmetic layered sheet comprising:
a hydrophilic substrate;
a thin film sheet having liquid permeability and laminated on the hydrophilic substrate; and
a support laminated on the thin film sheet, wherein
the thin film sheet is impregnated with a liquid containing a hydrophilic component, a lipophilic component, and a surfactant having an HLB value of 3 or more, and
the hydrophilic substrate has a contact angle with water smaller than that of a material constituting the support with water.

2. A cosmetic set comprising:
the cosmetic layered sheet according to Claim 1; and
a container arranged adjacent to the hydrophilic substrate and/or the support of the cosmetic layered sheet.

3. A cosmetic set comprising:
the cosmetic layered sheet according to Claim 1; and
a protection member arranged so as to face the hydrophilic substrate with the thin film sheet and the support of the cosmetic layered sheet therebetween, wherein
the hydrophilic substrate and the protection member are joined to each other so as to surround the thin film sheet and the support.

4. A method for manufacturing a cosmetic layered sheet, comprising:
a step of preparing a layered product in which a thin film sheet having liquid permeability and a support are laminated;
a step of preparing a hydrophilic substrate having a contact angle with water smaller than that of a material constituting the support with water, and laminating the layered product and the hydrophilic substrate in such a manner that the hydrophilic substrate faces the thin film sheet; and
a step of applying a liquid containing a hydrophilic component, a lipophilic component, and a surfactant having an HLB value of 3 or more onto the support.

5. A method for manufacturing a cosmetic layered sheet, comprising:
a step of preparing a layered product in which a thin film sheet having liquid permeability and a support are laminated;
a step of preparing a hydrophilic substrate having a contact angle with water smaller than that of a material constituting the support with water, and applying a liquid containing a hydrophilic component, a lipophilic component, and a surfactant having an HLB value of 3 or more onto at least one surface of the support; and
a step of laminating the layered product and the hydrophilic substrate in such a manner that the surface coated with the liquid of the hydrophilic substrate faces the thin film sheet.

6. A method for attaching a thin film sheet, comprising:
a support peeling step of peeling off the support from the cosmetic layered sheet according to Claim 1 to expose the thin film sheet; and
a thin film sheet attaching step of stacking the thin film sheet to a surface for attachment and then peeling off the hydrophilic substrate from the thin film sheet.

7. The method for attaching a thin film sheet according to Claim 6, further comprising:
a water application step of spraying water to a surface of the thin film sheet or a surface for adhesion after the support peeling step and before the thin film sheet attaching step.
